# EUROPEAN PATENT SPECIFICATION

(11) **EP 1 758 553 B1**
(45) Date of publication and mention of the grant of the patent: **24.02.2010**
(21) Application number: 05750655.2
(22) Date of filing: 08.06.2005
(51) Int. Cl.: A61K 9/00, A61K 47/34, A61K 47/10, A61K 38/10

(54) **PHARMACEUTICAL COMPOSITIONS COMPRISING POLYETHYLENE GLYCOL HAVING A MOLECULAR WEIGHT OF LESS THAN 600 DALTONS**
PHARMAZEUTISCHE ZUSAMMENSETZUNGEN AUS EINEM POLYETHYLENGLYKOL MIT EINEM MOLEKULARGEWICHT UNTER 600 DALTON
COMPOSITIONS PHARMACEUTIQUES COMPRENANT UN POLYETHYLENE GLYCOL DE POIDS INFERIEUR A 600 DALTONS

(30) Priority: 09.06.2004 GB 0412866
(43) Date of publication of application: 07.03.2007
(73) Proprietor: Novartis AG, 4056 Basel (CH); Novartis Pharma GmbH, 1230 Wien (AT)
(72) Inventor: LAMBERT, Olivier, F-68720 Spechbach-le-Haut (FR)
(74) Representative: Bohmann, Christine
(86) International application number: PCT/EP2005/006173
(87) International publication number: WO 2005/120453

(56) References cited:
- WO-A-01/45742
- WO-A-02/10192
- US-A- 5 192 741

## Description

The present invention relates to liquid pharmaceutical compositions, in particular to depot formulations comprising a pharmaceutically active agent and to a process for preparing said depot formulations. The active agent is cyclo[{4-(NH₂-C₂H₄-NH-CO-O-)Pro}-Phg-DTrp-Lys-Tyr(4-Bzl)-Phe]pamoate or di-aspartate

Depot formulations are typically administered parenterally. The active agent in liquid form may be administered by injection subcutaneously or intramuscularly through a small gauge needle or placed into accessible tissue sites through a cannula. However parenteral administration may be very painful especially if repeated injections are necessary. Furthermore, there may be difficulties with depot formulations which are administered in liquid form comprising more than 50 % of an organic solvent and which form a solid implant in the body after injection. Often the solidifying process starts in the syringe before injection and causes needle clogging. Depot formulations which form implants after injection may comprise a polymer or a mixture of polymers. These polymers have to be dissolved in an organic solvent. If the organic solvent remains in the solution for injection it might cause severe tissue irritation or necrosis at the site of implantation.

A variety of approaches have been developed to provide processes for preparing depot formulations. However these processes are often very complex comprising many different steps. Several processes include a heating step which might cause severe stability problems, e.g. of the excipients of the depot formulation such as polymers.

There is a need to provide improved depot formulations and simplified processes to prepare said depot formulations to overcome the above mentioned difficulties.

Surprisingly it has been found that advantageous parenteral depot formulations with a biodegradable polymer may be obtained if the composition comprises polyethylene glycol (PEG) with a molecular weight of less than 600 Daltons and less than about 0.5% of any other organic solvent.

The present invention provides in one aspect a liquid composition according to claim 1.

The composition of the invention may be stored e.g. in prefilled syringe over an extended period of time without precipitation. Further, the compositions of the invention are well tolerated, e.g. may show only negligible irritating, necrotic or toxic effects.

The depot formulations of the present invention are adapted to release all or substantially all the active agent over an extended period of time.

Also described is a process for preparing a depot formulation comprising the steps:
(i) dissolving a biodegradable polymer in a pharmaceutically acceptable organic solvent
(ii) optionally adding an additive to the polymer/solvent solution of step (i)
(iii) dissolving a pharmaceutically active agent in polyethylene glycol with a molecular weight of less than 600 Daltons
(iv) mixing solution (i) and (iii) or (ii) and (iii)
(v) removing the pharmaceutically acceptable organic solvent from the mixture e.g. by stirring and N₂ urging under reduced pressure or by tangential cross-flow filtration to afford dia-filtration.

Further described is a process for preparing a depot formulation comprising the steps:
(i) dissolving a biodegradable polymer in a pharmaceutically acceptable organic solvent
(ii) optionally mixing an additive with polyethylene glycol with a molecular weight of less than 600 Daltons.
(iii) dissolving a pharmaceutically active agent in polyethylene glycol with a molecular weight of less than 600 Daltons or in the polyethylene glycol/additive mixture of step (ii)
(iv) mixing solution (i) and (iii)
(v) removing the pharmaceutically acceptable organic solvent from the mixture e.g by stirring and N₂ urging under reduced pressure or by tangential cross-flow filtration to afford dia-filtration.

According to the invention, a pharmaceutically acceptable, organic solvent is used to dissolve the biodegradable polymer but this solvent is removed at the end of the process. The resulting compositions of the invention contain only minor amounts of organic solvent, e.g. irritating solvent, e.g. less than 0.5 % by weight based on the total weight of the composition.

As used herein, "biodegradable" means a material that should degrade by bodily processes to products readily disposable by the body and should not accumulate in the body.
Described polymers include
(a) linear or branched polyesters which are linear chains radiating from a polyol moiety, e.g. glucose,
(b) polyesters such as D-, L- or racemic polylactic acid, polyglycolic acid, polyhydroxybutyric acid, polycaprolactone, polyalkylene oxalate, polyalkylene glycol esters of acids of the Kreb's cycle, e.g. citric acid cycle, and the like and combinations thereof,
(c) polymers of organic ethers, anhydrides, amides, and orthoesters,
(d) copolymers of organic esters, ethers, anhydrides, amides, and orthoesters by themselves or in combination with other monomers.

The polymers may be cross-linked or non-cross-linked. Usually not more than 5%, typically less than 1 % are cross-linked.

The polymers of this invention are linear polyesters, and branched chain polyesters. The linear polyesters are prepared from lactic acid and glycolic acid, by condensation of the lactone dimers, see e.g. US 3,773,919. The polyester chains in the linear or branched (star) polymers are copolymers of lactic acid and glycolic acid. The molar ratios of lactide: glycolide of polylactide-co-glycolides preferably used according to the invention is preferably from about 95:5 to 5:95, e.g. 75:25 to 25:75, e.g. 60:40 to 40:60, with from 55:45 to 45:55, e.g. 52:48 to 48:52, e.g. 50:50.

Linear polyesters, e.g. linear polylactide-co-glycolides (PLG), preferably used according to the invention have a weight average molecular weight (Mw) between about 1,000 and about 50,000 Da, e.g. about 10,000 Da, and a polydispersity M_{w}/Mₙ e.g. between 1.2 and 2. The intrinsic viscosities of linear polymers of Mw 1000 to 50,000 are 0.05 to 0.6 dl/g, in chloroform. Suitable examples include e.g. those commonly known and commercially available as Resomers® from Boehringer Ingelheim, in particular Resomer® RG, e.g. Resomer® RG 502, 502H, 503, 503H, 504, 504H.

Branched polyesters, e.g. branched polylactide-co-glycolides, used according to the invention may be prepared using polyhydroxy compounds e.g. polyol e.g. glucose or mannitol as the initiator. These esters of a polyol are known and described e.g. in GB 2,145,422 B. The polyol contains at least 3 hydroxy groups and has a molecular weight of up to 20,000 Da, with at least 1, preferably at least 2, e.g. as a mean 3 of the hydroxy groups of the polyol being in the form of ester groups, which contain poly-lactide or co-poly-lactide chains. Typically 0.2% glucose is used to initiate polymerization. The branched polyesters (Glu-PLG) have a central glucose moiety having rays of linear polylactide chains, e.g. they have a star shaped structure.

The branched polyesters having a central glucose moiety having rays of linear polylactide-co-glycolide chains (Glu-PLG) may be prepared by reacting a polyol with a lactide and preferably also a glycolide at an elevated temperature in the presence of a catalyst, which makes a ring opening polymerization feasible.

The branched polyesters having a central glucose moiety having rays of linear polylactide-co-glycolide chains (Glu-PLG) preferably have a weight average molecular weight M_{w} in the range of from about 1,000 to 55,000, preferably 20,000, e.g. 10,000 Da, and a polydispersity e.g. of from 1.1 to 3.0, e.g. 2.0 to 2.5. The intrinsic viscosities of star polymers of M_{w} 10,000 to M_{w} 50,000 are 0.05 to 0.6 dl/g in chloroform. A star polymer having a M_{w} of 50,000 has a viscosity of 0.5 dl/g in chloroform.

The desired rate of degradation of polymers and the desired release profile for compounds of the invention may be varied depending on the kind of monomer, whether a homo- or a copolymer or whether a mixture of polymers is employed.

A mixture of polymers may comprise at least two different kinds of polymers, e.g. as listed under (a) to (e) above, or two polymers of the same polymer class with different properties. For example, a mixture of polymers may comprise a polymer having a medium weight average molecular weight, e.g. from about 30,000 to about 50,000 Da, e.g. of about 20,000 Da, and of a polymer having a low weight average molecular weight, e.g. of about 2.000 to about 20,000 Da, e.g. of about 10,000 Da.

Preferably, the polymer matrix comprises a linear and/or branched polylactide-co-glycolide. More preferably, the polymer matrix comprises a Resomer® RG and/or a star polylactide-co-glycolide polymer having a weight average molecular weight of about 10,000 Da and/or a star polylactide-co-glycolide polymer having a weight average molecular weight of about 50,000 Da. The ratio of linear to branched polylactide-co-glycolide preferably is 0 : 100 to 100 : 0, e.g. 50: 50 to 25: 75.

The solvent of the present invention may be miscible with polyethylene glycol. Examples of such solvents include N-methyl-2-pyrrolidone, 2-pyrrolidone, ethanol, acetone, acetonitrile, methyl acetate, methylene chloride, ethyl acetate, methyl ethyl ketone, dimethylformamide, dimethyl sulfoxide, tetrahydrofuran, caprolactam, decylmethylsulfoxide, oleic acid, and 1-dodecylazacycloheptan-2-one. Preferably acetone or methylene chloride may be used.

The amount of polymer dissolved in e.g. acetone or methylene chloride may be from about 10% w/v to about 40% w/v, preferably from about 15% w/v to about 30% w/v.

Optionally a an additive may be added to the polymer/solvent solution and/or to the polyethylene glycol/drug substance solution. The additive may improve the solubility of the polymer and the drug substance of the active ingredient. The co-solvent may further modulate the drug release in vitro or in vivo. The additive may be present in a amount of from about 0.1 % to about 20% w/v, preferably from about 1% to about 5%. Examples of such additives include methanol, ethanol, propylene glycol , liquid surfactant such as poly(oxyethylene) sorbitan esters (Tweens) or glycerin polyoxyethylene ester of castor oil (Cremophor EL), lactic acid, acetic acid, glycerol, N,N dimethylacetamide, benzyl benzoate, polyoxyethylated fatty acid, lecithin, soybean oil , seaflower oil, vegetable oils, cotton sead oils, oligormers of poly(I-lactide) of poly(d, I lactide) of poly(lactide co-glycolide) or a mixture of these oligomers.

The pharmaceutically active agent is dissolved or dispersed in liquid polyethylene glycols (PEG) having a molecular weight of less than 600 Daltons, e.g. PEG 200, PEG 300, PEG 400, PEG 540 or PEG 600 (Handbook of Pharmaceutical Excipients loc.cit., p. 454) or PEG with modified end groups e.g. polyethylene glycol mono and di-alkyl ether (Handbook of Pharmaceutical Excipients loc.cit. p. 469) or polyethylenglycol 600 mono and di-acid at room temperature, e.g. 25° C, e.g. depending on its solubility in this solvent with or without a co-solvent.

Details of suitable excipients for use in the process of the invention are described in the "Handbook of Pharmaceutical Excipients", Rowe, Sheskey and Weller, 4^{th} Edition 2003 which is incorporated by reference.

For the purpose of the invention "pharmaceutically active agent" means cyclo[{4-(NH₂-C₂H₄-NH-CO-O-)Pro}-Phg-DTrp-Lys-Tyr(4-Bzl)-Phe] (referred herein to as Compound A) as aspartate di-salt and pamoate monosalt, which is disclosed in WO 02/10192.

In a further aspect of the invention the composition obtainable by the process of the present invention is in liquid form, e.g. a solution. After sterile filtration through a 0.2 micrometer filter the liquid composition, e.g. solution, may be placed in a syringe. Sterilization may also be achieved by terminal sterilization with gamma irradiation at 20 to 30 kGy preferably at 25 kGy under cooled conditions, e.g. 2 to 8 °C or -70 °C. The sterilized solution may be injected through a needle, e.g. an up to 20 G needle, into the body subcutaneously or intramuscularly. Once in place the polyethylene glycol will dissipate and the polymer together with the pharmaceutically active agent solidifies to form the implant. Accordingly to the invention, preferably a prefilled syringe may be provided together with instructions for use.

In another aspect the invention provides a depot formulation for extended release of the pharmaceutically active agent. The implant formed after injection into the body may release the active agent over an extended period of time. The desired release profile may depend on the kind of monomer, whether a homo- or a co-polymer or whether a mixture of polymers is employed. The release period may range from 1 up to 12 weeks, e.g. 1 to 8 weeks.

The compositions of the invention are useful for treatment of the known indications of the particular active agent incorporated in the polymer. Compositions of the invention comprising a somatostatin anologue may be useful in the following indications:
a) for the prevention or treatment of disorders with an aetiology comprising or associated with excess GH-secretion and/or excess of IGF-1 e.g. in the treatment of acromegaly as well as in the treatment of type I or type II diabetes mellitus, especially complications thereof, e.g. angiopathy, diabetic proliferative retinopathy, diabetic macular edema, nephropathy, neuropathy and dawn phenomenon, and other metabolic disorders related to insulin or glucagon release, e.g. obesity, e.g. morbid obesity or hypothalamic or hyperinsulinemic obesity,
b) in the treatment of enterocutaneous and pancreaticocutaneous fistula, irritable bowel syndrom, inflammatory diseases, e.g. Grave's Disease, inflammatory bowel disease, psoriasis or rheumatoid arthritis, polycystic kidney disease, dumping syndrom, watery diarrhea syndrom, AIDS-related diarrhea, chemotherapy-induced diarrhea, acute or chronic pancreatitis and gastrointestinal hormone secreting tumors (e.g. GEP tumors, for example vipomas, glucagonomas, insulinomas, carcinoids and the like), lymphocyte malignancies, e.g. lymphomas or leukemias, hepatocellular carcinoma as well as gastrointestinal bleeding, e.g variceal oesophagial bleeding,
c) for the prevention or treatment of angiogenesis, inflammatory disorders as indicated above including inflammatory eye diseases, macular edema, e.g. cystoid macular edema, idiopathic cystoid macular edema, exudative age-related macular degeneration, choroidal neovascularization related disorders and proliferative retinopathy,
d) for preventing or combating graft vessel diseases, e.g. allo- or xenotransplant vasculopathies, e.g. graft vessel atherosclerosis, e.g. in a transplant of organ, e.g. heart, lung, combined heart-lung, liver, kidney or pancreatic transplants, or for preventing or treating vein graft stenosis, restenosis and/or vascular occlusion following vascular injury, e.g. caused by catherization procedures or vascular scraping procedures such as percutaneous transluminal angioplasty, laser treatment or other invasive procedures which disrupt the integrity of the vascular intima or endothelium,
e) for treating somatostatin receptor expressing or accumulating tumors such as pituitary tumors, e.g. Cushing's Disease, gastro-enteropancreatic, carcinoids, central nervous system, breast, prostatic (including advanced hormone-refractory prostate cancer), ovarian or colonic tumors, small cell lung cancer, malignant bowel obstruction, paragangliomas, kidney cancer, skin cancer, neuroblastomas, pheochromocytomas, medullary thyroid carcinomas, myelomas, lymphomas, Hodgkins and non-Hodgkins lymphomas, bone tumours and metastases thereof, as well as autoimmune or inflammatory disorders, e.g. rheumatoid arthritis, Graves disease or other inflammatory eye diseases.

Preferably, the compositions of the invention are useful in the treatment of acromegaly and cancer, e.g. Cushing's Disease.

The activity and the characteristics of the liquid compositions of the invention may be indicated in standard clinical or animal tests.
Appropriate dosage of the composition of the invention will of course vary, e.g. depending on the condition to be treated (for example the disease type of the nature of resistance), the drug used, the effect desired and the mode of administration.

For compositions of the invention comprising a somatostatin analogue satisfactory results are obtained on administration, e.g. parenteral administration, at dosages on the order of from about 0.2 to about 60 mg, preferably from about 5 to about 40 mg per injection per month or about 0.03 to about 1.2 mg per kg animal body weight per month, administered once or in divided doses. Suitable monthly dosages for patients are thus in the order of about 0.3 mg to about 40 mg of a somatostatin analogue, e.g. Compound A pamoate. The composition may be administered every 2 to 3 months. Suitable dosages for every 3 months administration are about 1 mg to about 180 mg.

Following is a description by way of example only of processes and compositions of the invention.

### Example 1

The solubility of different polymers (linear polymer): in polyethylene glycol is tested. The solubility of the linear polymers (Resomer®) and the star polymer (Poly(D),L-lactide-co-glycolide), D,L PLG-Glu is shown in table 1.

**Table 1**

| Polymer | Resomer® RG 50:50 Mw: 2100g/mol | Resomer® RG 502H Mw: 12000g/mol | Resomer® RG 502H | D,L-PLG-Glu Mw: 50800 g/mol |
|---|---|---|---|---|
| Polymer Concentration In PEG 300 | 10% | 10% | 20% | 10% |
| Appearance | Clear solution | Clear solution | Clear solution | Clear solution |

### Example 2

### Preparation of 20 ml polymer/PEG solution:

4.004 g Resomer RG 502 H are dissolved in 13.3 ml acetone. 20 ml polyethylene glycol PEG is added to this solution together with 25 mg/ml of Compound A pamoate. The complete solution is stirred 4 hours at room temperature and N₂ urging under reduced pressure. After sterile filtration the solution is filled in a syringe. The obtained prefilled syringe may be used for subcutaneous administration.

### Example 3

4 g of resomer RG502H were dissolved in 6.6 ml methylene chloride. 0.250 g Compound A di-aspartate were dissolved in 2 ml water and added to 20 ml PEG300. Both polymer and drug substance solutions were mixed together. The methylene chloride was evaporated for 5 hours at 40 °C in a water batch resulting in a injectable in situ forming depot formulation of 1.25 % w/v Compound A di-aspartate and 20% w/v Resomer RG502H in PEG 300.

### Example 4

1.5 g of resomer RG502H and 1.0 g of oligomers of poly(lactide-co-glycolide) 50:50 were dissolved in 3.3 ml methylene chloride. 0.250 g Compound A-pamoate were dissolved in 10 ml PEG300. Both polymer and drug substance solutions were mixed together. The methylene chloride was evaporated for 5 hours at 40 °C in a water batch resulting in a injectable in situ forming depot formulation of 2.5 % w/v Compound A pamoate and 15% w/v Resomer RG502H and 10% oligomers in PEG 300.

### Example 5

1.54 g of resomer RG502H and 0.5 g of benzyl benzoate were dissolved in 3.3 ml methylene chloride. 0.250 g Compound A-pamoate were dissolved in 10 ml PEG300. Both polymer and drug substance solutions were mixed together. The methylene chloride was evaporated for 5 hours at 40 °C in a water batch resulting in a injectable in situ forming depot formulation of 2.5 % w/v Compound A pamoate and 15% w/v Resomer RG502H and 5% benzyl benzoate in PEG 300.

### Example 6:

2.0 g of resomer RG502H were dissolved in 3.3 ml methylene chloride. 0.250 g Compound A-pamoate were dissolved in 10 ml PEG250 diethylether. Both polymer and drug substance solutions were mixed together. The methylene chloride was evaporated for 5 hours at 40 °C in a water batch resulting in a injectable in situ forming depot formulation of 2.5 % w/v Compound A pamoate and 20% w/v Resomer RG502H in PEG 250 diethylether.

### Example 7

4 g of resomer RG502H were dissolved in 6.6 ml methylene chloride. 0.50 g Compound A pamoate were dissolved in 20 ml PEG300. Both polymer and drug substance solutions were mixed together. The methylene chloride was evaporated for 5 hours at 40 °C in a water batch resulting in a injectable in situ forming depot formulation of 2.5 % w/v Compound A pamoate and 20% w/v Resomer RG502H in PEG 300.

## Claims

1. A liquid pharmaceutical composition comprising
(a) a linear or branched polylactide-co-glycolide as biodegradable polymer
(b) a polyethylene glycol having a molecular weight of less than 600 Daltons
(c) cyclo[{4-(NH₂-C₂H₄-NH-CO-O-)Pro}-Phg-DTrp-Lys-Tyr(4-Bzl)-Phe] pamoate or di-aspartate as pharmaceutically active agent, and
(d) less than about 0.5% of a pharmaceutically acceptable organic solvent, and optionally
(e) an additive

2. The liquid pharmaceutical composition according to claim 1 wherein the polyethylene glycol is PEG 200, PEG 300, PEG 400 or PEG 600 or di-alkyl ether PEG.

3. The liquid pharmaceutical composition according claim 1 or 2 wherein the pharmaceutically acceptable organic solvent is chosen from N-methyl-2-pyrrolidone, 2-pyrrolidone, ethanol, acetone, acetonitrile, methyl acetate, ethyl acetate, methyl ethyl ketone, methylene chloride, dimethylformamide, dimethyl sulfoxide, tetrahydrofuran, caprolactam, decylmehylsulfoxide, oleic acid, and 1-dodecylazacycloheptan-2-one.

4. The liquid pharmaceutical composition according to any preceding claim wherein the additive is choosen from methanol, ethanol, propylene glycol , liquid surfactant such as poly(oxyethylene) sorbitan esters (Tweens) or glycerin polyoxyethylene ester of ricin oil (Cremophore EL), lactic acid, acetic acid, glycerol, N,N dimethylacetamide, benzyl benzoate, polyoxyethylated fatty acid, lecithin, soybean oil , seaflower oil, vegetable oils, cotton sead oils, oligormers of poly(I-lactide) of poly(d,l lactide) of poly(lactide co-glycolide) or a mixture of these oligomers.

5. A process for preparing a depot formulation comprising the steps:
i) dissolving a linear or branched polylactide-co-glycolide as biodegradable polymer in a pharmaceutically acceptable organic solvent
ii) optionally adding an additive to the polymer/solvent solution of i)
iii) dissolving cyclo[{4-(NH₂-C₂H₄-NH-CO-O-)Pro}-Phg-DTrp-Lys-Tyr(4-Bzl)-Phe] pamoate or di-aspartate as pharmaceutically active agent in polyethylene glycol having a molecular weight of less than 600 Daltons
iv) mixing solution (i) and (iii) or (ii) and (iii)
v) removing the pharmaceutically acceptable organic solvent from the mixture.

6. The process according to claim 5 wherein optionally an additive is added to the polyethylene glycol before dissolving the pharmaceutically active agent in this solution.

7. The process according to claim 5 wherein at least two different polymers are dissolved in a pharmaceutically acceptable organic solvent.

8. The process according to any one of claims 5 to 7 wherein the polyethylene glycol is PEG 200, PEG 300, PEG 400 or PEG 600 or di-alkyl ether PEG.

9. The process according to any one of claims 5 to 8 wherein the pharmaceutically acceptable organic solvent is chosen from N-methyl-2-pyrrolidone, 2-pyrrolidone, ethanol, acetone, acetonitrile, methyl acetate, ethyl acetate, methyl ethyl ketone, methylen chloride, dimethylformamide, dimethyl sulfoxide, tetrahydrofuran, caprolactam, decylmehylsulfoxide, oleic acid, and 1-dodecylazacycloheptan-2-one.

10. A pharmaceutical composition comprising a pharmaceutically active agent wherein the composition is obtainable by the process according to any one of claims 5 to 9.

11. The pharmaceutical composition according to any one of claims 1 to 4 or 10 for injection.

12. The pharmaceutical composition according to any one of claims 1 to 4 or 10 to 11 which forms a solidified implant in the body after injection.

13. The pharmaceutical composition according to any one of claims 1 to 4 or 10 to 12 wherein the active agent is released over 1 up to 12 weeks.

14. A prefilled syringe comprising the composition of any one of claims 1 to 4 or 10 to 13 and instructions to use.

## Patentansprüche

1. Flüssige pharmazeutische Zusammensetzung, die
(a) ein lineares oder verzweigtes Polylactid-co-glycolid als biologisch abbaubares Polymer,
(b) ein Polyethlyenglycol mit einem Molekulargewicht von weniger als 600 Dalton,
(c) Cyclo[{4-(NH₂-C₂H₄-NH-CO-O-)Pro}-Phg-DTrp-Lys-Tyr(4-Bzl)-Phe]-pamoat oder -diaspartat als pharmazeutisch wirksames Mittel und
(d) weniger als etwa 0.5 % eines pharmazeutisch akzeptablen organischen Lösemittels und optional
(e) ein Additiv
umfasst.

2. Flüssige pharmazeutische Zusammensetzung nach Anspruch 1, wobei das Polyethylenglycol PEG 200, PEG 300, PEG 400 oder PEG 600 oder ein Dialkylether-PEG ist.

3. Flüssige pharmazeutische Zusammensetzung nach Anspruch 1 oder 2, wobei das pharmazeutisch akzeptable organische Lösemittel aus N-Methyl-2-pyrrolidon, 2-Pyrrolidon, Ethanol, Aceton, Acetonitril, Methylacetat, Ethylacetat, Methylethylketon, Methylenchlorid, Dimethylformamid, Dimethylsulfoxid, Tetrahydrofuran, Caprolactam, Decylmethylsulfoxid, Ölsäure und 1-Dodecylazacycloheptan-2-on ausgewählt ist.

4. Flüssige pharmazeutische Zusammensetzung nach einem der vorhergehenden Ansprüche, wobei das Additiv aus Methanol, Ethanol, Propylenglycol, flüssigem grenzflächenaktivem Mittel, wie Poly(oxyethylen)sorbitanestern (Tween) oder Glycerinpolyoxyethylenester von Rizinusöl (Cremophore EL), Milchsäure, Essigsäure, Glycerin, N,N-Dimethylacetamid, Benzylbenzoat, polyoxyethylierter Fettsäure, Lecithin, Sojaöl, Sonnenblumenöl, pflanzlichen Olen, Baumwollölen, Oligomeren von Poly(l-lactid) von Poly(d, I-lactid) von Poly(lactid-co-glycolid) oder einem Gemisch dieser Oligomere ausgewählt ist.

5. Verfahren zur Herstellung einer Depotformulierung, das die folgenden Stufen umfasst:
i) Lösen eines linearen oder verzweigten Polylactid-co-glycolids als biologisch abbaubaren Polymer in einem pharmazeutisch akzeptablen organischen Lösemittel,
ii) optionales Zugeben eines Additivs zu der Polymer/Lösemittel-Lösung von i),
iii) Auflosen von Cyclo[{4-{NH₂-C₂H₄-NH-CO-O-)Pro}-Phg-DTrp-Lys-Tyr(4-Bzl)-Phe]-pamoat oder -diaspartat als pharmazeutisch wirksamem Mittel in Polyethylenglycol mit einem Molekulargewicht von weniger als 600 Dalton,
iv) Mischen der Lösungen (i) und (iii) oder(ii) und (iii),
v) Entfernten des pharmazeutisch akzeptablen organischen Lösemittels aus dem Gemisch.

6. Verfahren nach Anspruch 5, wobei optional ein Additiv zu dem Polyethylenglycol vor einem Auflösen des pharmazeutisch wirksamen Mittels in dieser Lösung zugegeben wird.

7. Verfahren nach Anspruch 5, wobei mindestens zwei unterschiedliche Polymere in einem pharmazeutisch akzeptablen organischen Lösemittel gelöst werden.

8. Verfahren nach einem der Ansprüche 5 bis 7, wobei das Polyethylenglycol PEG 200, PEG 300, PEG 400 oder PEG 600 oder Dialkylether-PEG ist.

9. Verfahren nach einem der Ansprüche 5 bis 8, wobei das pharmazeutisch akzeptable organische Lösemittel aus N-Methyl-2-pyrrolidon, 2-pyrrolidon, Ethanol, Aceton, Acetonitril, Methylacetat, Ethylacetat, Methylethylketon, Methylenchlorid, Dimethylformamid, Dimethylsulfoxid, Tetrahydrofuran, Caprolactam, Decylmethylsulfoxid, Ölsäure und 1-Dodecylazacycloheptan-2-on ausgewählt ist.

10. Pharmazeutische Zusammensetzung, die ein pharmazeutisch wirksames Mittel umfasst, wobei die Zusammensetzung nach dem Verfahren gemäß einem der Ansprüche 5 bis 9 erhältlich ist.

11. Pharmazeutische Zusammensetzung nach einem der Ansprüche 1 bis 4 oder 10 für eine Injektion.

12. Pharmazeutische Zusammensetzung nach einem der Ansprüche 1 bis 4 oder 10 bis 11, die im Körper nach einer Injektion ein verfestigtes implantat bildet.

13. Pharmazeutische Zusammensetzung nach einem der Ansprüche 1 bis 4 oder 10 bis 12, wobei das wirksame Mittel über 1 bis zu 12 Wochen freigesetzt wird.

14. Vorgefüllte Spritze, die die Zusammensetzung nach einem der Ansprüche 1 bis 4 oder 10 bis 13 und Instruktionen zur Verwendung umfasst.

## Revendications

1. Composition pharmaceutique liquide comprenant:
(a) un polylactide-co-glycolide linéaire ou ramifié en tant que polymère biodégradable,
(b) un polyéthylèneglycol ayant un poids moléculaire inférieur à 600 Daltons,
(c) le pamoate ou le diaspartate de cyclo[{4-(NH₂-C₂H₄-NH-CO-O)Pro}-Phg-DTrp-Lys-Tyr(4-Bzl)-Phe] en tant qu'agent actif sur le plan pharmaceutique, et
(d) moins d'environ 0,5% d'un solvant organique acceptable sur le plan pharmaceutique, et facultativement
(e) un additif.

2. Composition pharmaceutique liquide selon la revendication 1, dans laquelle le polyéthylèneglycol est le PEG 200, le PEG 300, le PEG 400 ou le PEG 600 ou un éther dialkylique de PEG.

3. Composition pharmaceutique liquide selon la revendication 1 ou 2, dans laquelle le solvant organique acceptable sur le plan pharmaceutique est choisi parmi la N-méthyl-2-pyrrolidone, la 2-pyrrolidone, l'éthanol, l'acétone, l'acétonitrile, l'acétate de méthyle, l'acétate d'éthyle, la méthyléthylcétone, le chlorure de méthylène, le diméthylformamide, le diméthylsulfoxyde, le tétrahydrofuranne, le caprolactame, le décylméthylsulfoxyde, l'acide oléique et la 1-dodécylazacycloheptan-2-one.

4. Composition pharmaceutique liquide selon l'une quelconque des revendications précédentes, dans laquelle l'additif est choisi parmi le méthanol, l'éthanol, le propylèneglycol, un agent tensioactif liquide tel que les esters de poly(oxyéthylène)sorbitane (Tweens) ou l'ester de glycérine-polyoxyéthylène d'huile de ricin (Cremophor EL), l'acide lactique, l'acide acétique, le glycérol, le N,N-diméthylacétamide, le benzoate de benzyle, l'acide gras polyoxyéthylé, la lécithine, l'huile de soja, l'huile d'actinie, les huiles végétales, les huiles de graines de coton, les oligomères de poly(L-lactide) de poly(D,L-lactide) de poly(lactide-co-glycolide) ou un mélange de ces oligomères.

5. Procédé de préparation d'une formulation retard, comprenant les étapes consistant à:
i) dissoudre un polylactide-co-glycolide linéaire ou ramifié en tant que polymère biodégradable dans un solvant organique acceptable sur le plan pharmaceutique,
ii) ajouter facultativement un additif à la solution de polymère/solvant du point i),
iii) dissoudre le pamoate ou le diaspartate de cyclo[{4-(NH₂-C₂H₄-NH-CO-O)Pro}-Phg-DTrp-Lys-Tyr(4-Bzl)-Phe] en tant qu'agent actif sur le plan pharmaceutique dans du polyéthylèneglycol ayant un poids moléculaire inférieur à 600 Daltons,
iv) mélanger (i) et (iii) ou (ii) et (iii),
v) éliminer du mélange le solvant organique acceptable sur le plan pharmaceutique.

6. Procédé selon la revendication 5, dans lequel un additif est ajouté facultativement au polyéthylèneglycol avant de dissoudre l'agent actif sur le plan pharmaceutique dans cette solution.

7. Procédé selon la revendication 5, dans lequel au moins deux polymères différents sont dissous dans un solvant organique acceptable sur le plan pharmaceutique.

8. Procédé selon l'une quelconque des revendications 5 à 7, dans lequel le polyéthylèneglycol est le PEG 200, le PEG 300, le PEG 400 ou le PEG 600 ou un éther dialkylique de PEG.

9. Procédé selon l'une quelconque des revendications 5 à 8, dans lequel le solvant organique acceptable sur le plan pharmaceutique est choisi parmi la N-méthyl-2-pyrrolidone, la 2-pyrrolidone, l'éthanol, l'acétone, l'acétonitrile, l'acétate de méthyle, l'acétate d'éthyle, la méthyléthylcétone, le chlorure de méthylène, le diméthylformamide, le diméthylsulfoxyde, le tétrahydrofuranne, le caprolactame, le décylméthylsulfoxyde, l'acide oléique, et la 1-dodécylazacycloheptan-2-one.

10. Composition pharmaceutique comprenant un agent actif sur le plan pharmaceutique, où la composition peut être obtenue par le procédé selon l'une quelconque des revendications 5 à 9.

11. Composition pharmaceutique selon l'une quelconque des revendications 1 à 4 ou 10, destinée à l'injection.

12. Composition pharmaceutique selon l'une quelconque des revendications. 1 à 4 ou 10 à 11, qui forme un implant solidifié dans l'organisme après l'injection.

13. Composition pharmaceutique selon l'une quelconque des revendications 1à 4 ou 10 à 12, dans laquelle l'agent actif est libéré sur une période de 1 jusqu'à 12 semaines.

14. Seringue préremplie comprenant la composition, selon l'une quelconque des revendications 1 à 4 ou 10 à 13 et le mode d'emploi.
